(19) **Europäisches Patentamt**
**European Patent Office**
**Office européen des brevets**

(11) **EP 4 425 499 A1**

(12) **EUROPEAN PATENT APPLICATION**
published in accordance with Art. 153(4) EPC

(43) Date of publication:
04.09.2024 Bulletin 2024/36

(21) Application number: 22887598.5

(22) Date of filing: 26.10.2022

(51) International Patent Classification (IPC):
*G16B 5/00* (2019.01)     *G16B 35/10* (2019.01)
*G16B 30/10* (2019.01)    *G16B 40/00* (2019.01)
*G06N 3/04* (2023.01)     *G06N 3/08* (2023.01)
*C12Q 1/6886* (2018.01)

(52) Cooperative Patent Classification (CPC):
C12Q 1/6886; G06N 3/04; G06N 3/08; G16B 5/00;
G16B 30/10; G16B 35/10; G16B 40/00

(86) International application number:
PCT/KR2022/016448

(87) International publication number:
WO 2023/075402 (04.05.2023 Gazette 2023/18)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 26.10.2021 KR 20210143610

(71) Applicant: GC Genome Corporation
Yongin-si, Gyeonggi-do 16924 (KR)

(72) Inventors:
• CHO, Eun-Hae
Yongin-si Gyeonggi-do 16924 (KR)
• AHN, Jin Mo
Yongin-si Gyeonggi-do 16924 (KR)
• KI, Chang-Seok
Yongin-si Gyeonggi-do 16924 (KR)
• LEE, Junnam
Yongin-si Gyeonggi-do 16924 (KR)

(74) Representative: Mewburn Ellis LLP
Aurora Building
Counterslip
Bristol BS1 6BX (GB)

(54) **METHOD FOR DIAGNOSIS OF CANCER AND PREDICTION OF CANCER TYPE, USING METHYLATED ACELLULAR NUCLEIC ACID**

(57) The present invention relates to a method for diagnosing cancer and predicting cancer types using a methylated cell-free nucleic acid, and more particularly, to a method for diagnosing cancer and predicting cancer types using a method for extracting methylated nucleic acids from a biospecimen, generating vectorized data of nucleic acid fragments based on aligned reads by obtaining sequence information, and then inputting the data into a trained artificial intelligence model so as to analyze a calculated value. The method for diagnosing cancer and predicting cancer types using methylated cell-free nucleic acids according to the present invention is useful because it generates vectorized data and analyzes it using an AI algorithm, compared to methods that use a conventional step of determining the amount of chromosomes based on the read count or detection methods that use the concept of distance between aligned reads to utilize values related to reads as one by one structured values, so that a similar effect can be achieved even if the read coverage is low.

EP 4 425 499 A1

**FIG. 1**

Concentrating a methylated cell-free nucleic acid

Acquiring concentrated nucleic acid fragments by using massively parallel sequencing method

Aligning fragment data to human reference chromosome

Determining quality of aligned data

Generating a GC (Grand Canyon) plot

Training CNN (convolution neural network)

Calculating deep probability index (DPI)

Predicting whether the subject is a cancer patient and the type of cancer

**Description**

**Technical Field**

The present invention relates to a method for diagnosing cancer and predicting cancer type using methylated cell-free nucleic acids, and more specifically, to a method for diagnosing cancer and predicting cancer type using a method for extracting nucleic acids from a biospecimen, obtaining sequence information including methylation information, generating vectorized data of nucleic acid fragments based on aligned reads, and inputting the data into a trained artificial intelligence model to analyze the calculated values.

**Related Art**

The diagnosis of cancer in clinical practice is usually confirmed by case history study, physical examination, and clinical evaluation, followed by a tissue biopsy. A clinical diagnosis of cancer is only possible when the number of cancer cells is more than one billion and the diameter of the cancer is more than one centimeter. In this case, the cancer cells already have the ability to metastasize, and at least half of them have already metastasized. In addition, biopsies are invasive, causing considerable discomfort to the patient, and it is often not possible to perform a biopsy while treating a cancer patient. In addition, tumor markers are used in cancer screening to monitor substances produced directly or indirectly by cancer, but their accuracy is limited because more than half of tumor marker screening results are normal even in the presence of cancer, and they are often positive even in the absence of cancer.

In response to the need for a relatively simple, non-invasive, and highly sensitive and specific cancer diagnostic method that can compensate for the shortcomings of conventional cancer diagnostic methods, liquid biopsy, which utilizes a patient's body fluids to diagnose and follow up on cancer, has recently been widely used. Liquid biopsy is a non-invasive method that is drawing attention as an alternative to existing invasive diagnostic and testing methods.

Recently, methods for performing cancer diagnosis and cancer type differentiation by using cell free DNA obtained from liquid biopsy have been developed (US 10975431, Zhou, Xionghui et al., bioRxiv, 2020.07.16.201350), and in particular, methods for determining cancer diagnosis/type using methylation patterns of cell-free nucleic acids are known (Li, Jiaqi et al., bioRxiv, 2021.01.12.426440, US 2020-0131582, KR 10-2148547).

Meanwhile, an artificial neural network is a computational model implemented in software or hardware that mimics the computational power of a biological system by using a large number of artificial neurons connected by wires. Artificial neural networks use artificial neurons that simplify the functions of biological neurons. They are interconnected through connection lines with connection strengths to perform human cognition or learning processes. The connection strength is a specific value of the connection line, also known as the connection weight. The learning of an artificial neural network can be divided into supervised learning and unsupervised learning. Supervised learning is a method of inputting input data and corresponding output data into a neural network, and updating the connection strength of the connection lines so that the output data corresponding to the input data is output. Representative learning algorithms include Delta Rule and error back propagation learning. Unsupervised learning is a method in which an artificial neural network learns connection strengths by itself using only input data without a target value. Unsupervised learning is a method that updates the connection weights based on the correlation between input patterns.

Many data applied in machine learning suffer from the curse of dimensionality, which is that as the dimensionality of the required data goes to infinity, the distance between two arbitrary points diverges to infinity, and the amount of data present, or density, becomes somewhat lower in high-dimensional space, making it difficult to properly reflect the features of the data (Richard Bellman, Dynamic Programming, 2003, chapter 1). The recent development of deep learning, which is a structure with a hidden layer between the input layer and the output layer, has been reported to significantly improve the performance of classifiers in high-dimensional data such as images, videos, and signal data by processing the linear combination of variable values from the input layer into a nonlinear function (Hinton, Geoffrey, et al., IEEESignal Processing Magazine Vol. 29.6, pp. 82-97, 2012).

There are various patents (KR 10-2018-124550, KR 10-2019-7038076, KR 10-2019-0003676, KR 10-2019-0001741) that utilize these artificial neural networks for bio applications, and the inventors of the present invention have applied for a patent (KR 10-2021-0067931) on a method for detecting chromosomal abnormalities through artificial neural network analysis based on sequencing information of cell-free DNA (cfDNA) in blood.

However, no one has ever imaged and analyzed methylated cell-free nucleic acids and no one has ever represented methylation patterns on a whole-genome scale.

Accordingly, the inventors of the present invention have made good faith efforts to solve the above problems and develop an artificial intelligence-based cancer diagnosis method with high sensitivity and accuracy, and have confirmed that if vectorized data is generated based on the distance or amount of methylated cell-free nucleic acid fragments and analyzed with a trained artificial intelligence model, cancer diagnosis and cancer type identification can be performed with high sensitivity and accuracy, and have completed the present invention.

Summary of Invention

It is an object of the present invention to provide a method for diagnosing cancer and predicting cancer type using methylated cell-free nucleic acids.

Another object of the present invention is to provide a device for cancer diagnosis and cancer type prediction using methylated cell-free nucleic acids.

Another object of the present invention is to provide a computer-readable storage medium comprising instructions configured to be executed by a processor for diagnosing cancer and predicting cancer type by the above methods.

To achieve the above objectives, the present invention provides a method for providing information for diagnosing cancer and predicting cancer type, comprising the steps of (a) obtaining a sequence information including methylation information from extracted nucleic acids from a biospecimen; (b) aligning the obtained sequence information (reads) to a reference genome sequence database; (c) generating vectorized data using nucleic acid fragments based on the aligned sequence information (reads); (d) inputting the generated vectorized data into a trained artificial intelligence model and comparing the analyzed output value with a cut-off value to determine the presence or absence of cancer; and (e) predicting a cancer type through the comparison with the output result.

The present invention also provides a cancer diagnosis and cancer type prediction device comprising a decoding part that extracts nucleic acid from a biospecimen and decodes sequence information including methylation information; an alignment part that aligns the decoded sequence to a reference genome database; a data generation part that generates vectorized data using nucleic acid fragments based on the aligned sequence; a cancer diagnosis part that inputs the generated vectorized data into a trained artificial intelligence model, analyzes same, and determines the presence or absence of cancer by comparing the analyzed output value to a cut-off value; and a cancer type prediction part that analyzes the output result value to predict a cancer type.

The present invention also relates to a computer-readable storage medium comprising instructions configured to be executed by a processor for diagnosing cancer and predicting a cancer type, the instructions comprising (a) obtaining a sequence information including methylation information from extracted nucleic acids from a biospecimen; (b) aligning the obtained sequence information (reads) to a reference genome database; (c) generating vectorized data using nucleic acid fragments based on the aligned sequence information (reads); (d) inputting the generated vectorized data into a trained artificial intelligence model and comparing the analyzed output value with a cut-off value to determine the presence or absence of cancer; and (e) predicting a cancer type through the comparison with the output result.

The present invention provides a method for diagnosing cancer and predicting a cancer type, comprising the steps of (a) obtaining a sequence information including methylation information from extracted nucleic acids from a biospecimen; (b) aligning the obtained sequence information (reads) to a reference genome database; (c) generating vectorized data using nucleic acid fragments based on the aligned sequence information (reads); (d) inputting the generated vectorized data into a trained artificial intelligence model and comparing the analyzed output value with a cut-off value to determine the presence or absence of cancer; and (e) predicting a cancer type through the comparison with the output result.

**Brief Description of Drawing**

FIG. 1 is an overall flowchart of the present invention for determining chromosomal abnormalities based on artificial intelligence.

FIG. 2 is an example of a GC plot generated based on methylated cfDNA according to one embodiment of the present invention, wherein the x-axis is the chromosome per segment and the y-axis is the number of nucleic acid fragments between each segment.

FIG. 3 shows the accuracy of neuroblastoma determination for a deep learning model trained on GC plot image data generated based on the number of nucleic acid fragments using methylated cfDNA, according to one embodiment of the present invention.

FIG. 4 is a result showing the probability distribution for each data set of neuroblastoma determination for a deep learning model trained on GC plot image data generated based on the number of nucleic acid fragments using methylated cfDNA, according to one embodiment of the present invention, wherein (A) refers to a training set, (B) refers to a validation set, and (C) refers to a test set.

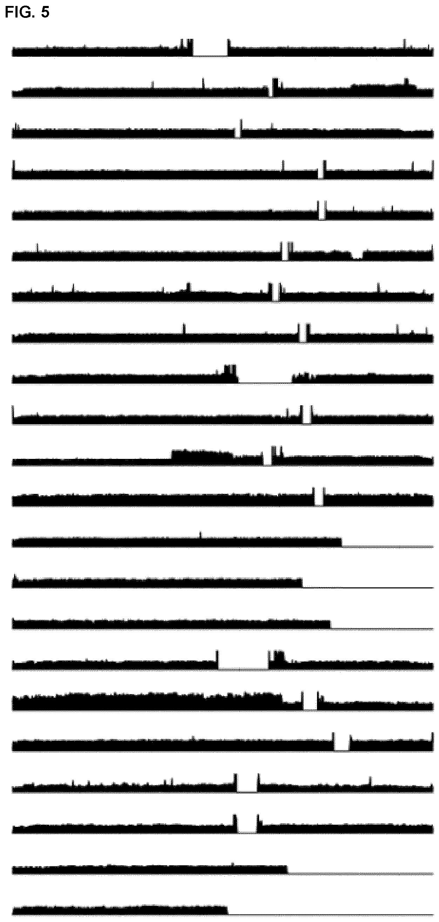

FIG. 5 is an example of a GC plot generated based on cfDNA in accordance with one embodiment of the present invention, wherein the x-axis is a chromosome per segment and the y-axis is the number of nucleic acid fragments between each segment.

FIG. 6 is a result of checking the accuracy of neuroblastoma determination for a deep learning model trained on GC plot image data generated based on the number of nucleic acid fragments using cfDNAs, according to one embodiment of the present invention.

FIG. 7 is a result showing a probability distribution for each data set of neuroblastoma determination for a deep learning model trained on GC plot image data generated based on the number of nucleic acid fragments using cfDNA, according to one embodiment of the present invention, wherein (A) means a training set, (B) means a validation set, and (C) means a test set.

## Detailed Description of the invention and Preferred Embodiments

Unless otherwise defined, all technical and scientific terms used herein have the same meaning as commonly understood by a person skilled in the art. In general, the nomenclature used herein is well known and in common use in the art.

The terms "first," "second," "A," "B," and the like may be used to describe various components, but the components are not limited by such terms and are used only to distinguish one component from another. For example, without departing from the scope of the technology described herein, a "first component" may be named a "second component," and similarly, a "second component" may be named a "first component." The term "and/or" includes any combination of a plurality of related recited components or any of a plurality of related recited components.

As used herein, the singular expression shall be understood to include the plural expression unless the context clearly indicates otherwise, and the term "comprising" shall be understood to mean the presence of the features, numbers, steps, actions, components, parts, or combinations thereof set forth, and not to exclude the possibility of the presence or addition of one or more other features, numbers, steps, actions components, parts, or combinations thereof.

Before proceeding to a detailed description of the drawings, it should be clarified that the division of the components herein is only by the primary function performed by each component, i.e., two or more of the components described herein may be combined into a single component, or a single component may be divided into two or more components with more detailed functions. Each of the components described herein may additionally perform some or all of the functions of other components in addition to their own primary functions, and some of the primary functions of each component may be dedicated and performed by other components.

Further, in performing the method or operation method, the steps comprised in the method may occur in a different order from that specified unless the context clearly indicates a particular order, i.e., the steps may occur in the same order as specified, may be performed substantially simultaneously, or may be performed in reverse order.

In this invention, it is sought to confirm that cancer can be detected with high sensitivity and accuracy by aligning sequencing data obtained from methylated cell-free nucleic acids extracted from a sample to a reference genome, generating vectorized data based on the aligned nucleic acid fragments, and then calculating the DPI value from a trained artificial intelligence model and comparing the analyzed data to a cut-off value.

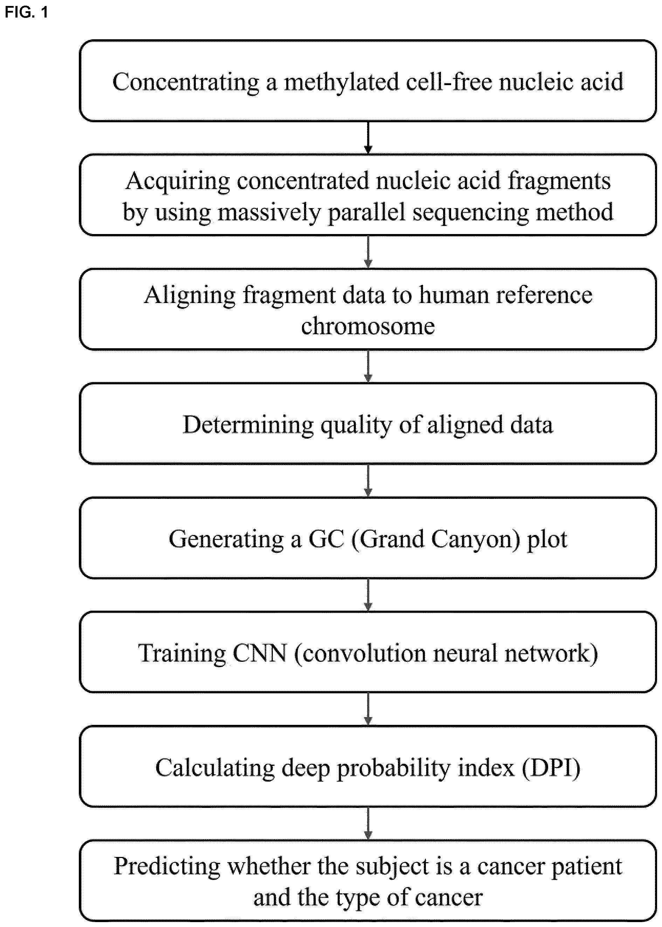

In other words, in one embodiment of the present invention, a method of sequencing DNA extracted from blood to include methylation information, aligning same to a reference genome, calculating the distance or amount between nucleic acid fragments for each chromosome segment, generating vectorized data with each genetic segment as the x-axis, and with the distance or amount between nucleic acid fragments as the y-axis and training same to a deep learning model to calculate the DPI value, and determining the presence of cancer if the DPI value is above a cut-off value, and determining the cancer type with the highest value among multiple DPI values as the actual cancer type (FIG. 1) was developed.

Accordingly, in one aspect, the present invention provides a method for providing information for diagnosing cancer and predicting cancer type, comprising the steps of:

(a) obtaining a sequence information including methylation information from extracted nucleic acids from a biospecimen;
(b) aligning the obtained sequence information (reads) to a reference genome database;
(c) generating vectorized data using nucleic acid fragments based on the aligned sequence information (reads);
(d) inputting the generated vectorized data into a trained artificial intelligence model and comparing the analyzed output value with a cut-off value to determine the presence or absence of cancer; and
(e) predicting a cancer type through the comparison with the output result.

In the present invention, the nucleic acid fragment may be any fragment of nucleic acid extracted from a biospecimen, but preferably, but not limited to, a fragment of cell-free nucleic acid or intracellular nucleic acid.

In the present invention, the nucleic acid fragment can be obtained by any method known to a person of ordinary skill in the art, preferably by direct sequencing, by sequencing by next-generation sequencing, non-specific whole genome amplification, or probe-based sequencing, but is not limited thereto.

In the present invention, the nucleic acid fragment may represent a read when utilizing next generation sequencing.

In the present invention, the cancer may be a solid or blood cancer, preferably may be selected from the group consisting of non-Hodgkin lymphoma, non-Hodgkin lymphoma, acute-myeloid leukemia, acute-lymphoid leukemia, multiple myeloma, head and neck cancer, lung cancer, glioblastoma, colon/rectal cancer, pancreatic cancer, breast cancer, ovarian cancer, melanoma, prostate cancer, liver cancer, thyroid cancer, stomach cancer, gallbladder cancer, biliary tract cancer, bladder cancer, small intestine cancer, cervical cancer, cancer of unknown primary site, kidney cancer, esophageal cancer, neuroblastoma and mesothelioma, and more preferably, neuroblastoma, but is not limited thereto.

In the present invention,

In the present invention, the step (a) comprises the steps of:

(a-i) obtaining nucleic acids with methylation information from a biospecimen;
(a-ii) removing proteins, fats, and other residues from the collected nucleic acid by using a salting-out method, a column chromatography method, or a beads method to obtain purified nucleic acid;
(a-iii) creating a single-end sequencing or pair-end sequencing library from purified nucleic acids or nucleic acids that have been randomly fragmented by enzymatic cleavage, grinding, or hydroshear methods;
(a-iv) reacting the created library to a next-generation sequencer; and
(a-v) acquiring nucleic acid sequence information (reads) from a next-generation sequencer.

In the present invention, the step (a) of obtaining the sequence information may be characterized by, but is not limited to, obtaining the isolated cell-free DNA by whole-genome sequencing to a depth of 1 million to 100 million reads.

In the present invention, a biospecimen means any substance, biological fluid, tissue or cell obtained from or derived from an individual, for example, may include whole blood, leukocytes, peripheral blood mononuclear cells, leukocyte buffy coat, blood (including plasma and serum), sputum, tears, mucus, nasal washes, nasal aspirate, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extracts, hair, oral cells, placental cells, cerebrospinal fluid, or mixtures thereof, but is not limited thereto.

As used in the present invention, the term "reference group" refers to a group of individuals who are currently free of a particular disease or condition, which is a reference group to which a comparison can be made, such as a standard sequence database. In the present invention, in a reference genome database of a reference group, the standard sequence may be a reference chromosome registered with a public health organization such as NCBI.

In the present invention, the nucleic acid in step (a) may be cell-free DNA, more preferably, circulating tumor cell DNA, but is not limited thereto.

In the present invention, the nucleic acids containing the methylation information can be obtained by various methods known in the art, preferably by bisulfite conversion, enzymatic conversion or methylated DNA immunoprecipitation (MeDIP), but is not limited thereto.

In the present invention, a method for detecting DNA methylation further includes a restriction enzyme-based detection method, wherein a methylation restriction enzyme (MRE) is used to cut unmethylated nucleic acids, or a specific sequence (recognition site), whether methylated or not, is cut and analyzed in combination with a hybridization method or PCR.

The methods based on bisulfite substitution in the present invention include Whole-Genome Bisulfite Sequencing (WGBS), Reduced-Representation Bisulfite Sequencing (RRBS), Methylated CpG Tandems Amplification and Sequencing (MCTA-seq), Targeted Bisulfite Sequencing, Methylation Array, and Methylation-specific PCR (MSP).

In the present invention, methods for enrichment and analysis of methylated DNA include Methylated DNA Immunoprecipitation Sequencing (MeDIP-seq), Methyl-CpG Binding Domain Protein Capture Sequencing (MBD-seq), and others.

Another method for analyzing methylated DNA in the present invention is 5-hydroxymethylation profiling, examples of which include 5hmC-Seal (hMe-Seal), hmC-CATCH, Hydroxymethylated DNA Immunoprecipitation Sequencing (hMeDIP-seq), and Oxidative Bisulfite Conversion.

In the present invention, the next-generation sequencer may be used with any sequencing method known in the art. Sequencing of the nucleic acids isolated by the selected method is typically performed by using next-generation sequencing (NGS). Next-generation sequencing includes any sequencing method that determines one nucleic acid sequence of an individual nucleic acid molecule or a clonally extended proxy for an individual nucleic acid molecule in a highly similar manner (e.g., 105 or more molecules are sequenced simultaneously). In one embodiment of the present invention, the relative abundance of a nucleic acid type in the library can be estimated by counting the relative number of occurrences of its homologous sequences in the data generated by the sequencing experiment. Next-generation sequencing methods are known in the art and are described, for example, in Metzker, M. (2010) Nature Biotechnology Reviews 11:31-46, which is incorporated herein by reference.

In one embodiment of the present invention, next-generation sequencing is used to determine the nucleic acid sequence of individual nucleic acid molecules (e.g., HeliScope Gene Sequencing system from Helicos BioSciences and PacBio RS system from Pacific Biosciences). In other embodiments, sequencing, for example, massively parallel, short-read sequencing that produces more bases of sequence per unit of sequencing than other sequencing methods that produce fewer but longer reads (e.g., Solexa sequencer from Illumina Inc. located in San Diego, CA) determines the nucleic acid sequence of a clonally extended proxy for an individual nucleic acid molecule (e.g., Solexa sequencer from Illumina Inc. located in San Diego, CA; 454 Life Sciences (Branford, CT) and Ion Torrent). Other methods or machines for next-generation sequencing are provided by, but not limited to, 454 Life Sciences (Branford, CT), Applied Biosystems (Foster City, CA; SOLiD sequencer), Helikos Bioscience Corporation (Cambridge, MA), and emulsion and microfluidic sequencing techniques nanodroplets (e.g., GnuBio droplets).

Platforms for next-generation sequencing include, but are not limited to, Roche/454's Genome Sequencer (GS) FLX system, Illumina/Solexa's Genome Analyzer (GA), Life/APG's Support Oligonucleotide Ligation Detection (SOLiD) system, Polonator's G.007 system, Helicos BioSciences' HeliScope Gene Sequencing system, Oxford Nanopore Technologies' PromethION, GriION, and MinION systems, and Pacific Biosciences' PacBio RS system.

In the present invention, the sequence alignment of step (b) comprises a computerized method or approach used for identity, wherein the read sequence (for example, from next-generation sequencing, e.g., a short-read sequence) in the genome is likely to be derived by evaluating the similarity between the read sequence and a reference sequence in most cases as a computer algorithm. A variety of algorithms can be applied to the sequence alignment problem. Some algorithms are relatively slow, but allow for relatively high specificity. These include, for example, dynamic programming-based algorithms. Dynamic programming is a method of solving complex problems by breaking them down into simpler steps. Other approaches are relatively more efficient, but typically not as thorough. These include, for example, heuristic algorithms and probabilistic methods designed for searching large databases.

Typically, there are two steps in the alignment process: candidate screening and sequence alignment. Candidate screening reduces the search space for sequence alignment from the entire genome for a shorter enumeration of possible alignment positions. Sequence alignment, as the term suggests, involves aligning sequences with sequences provided in the candidate screening step. This can be done using a global alignment (e.g., Needleman-Wunsch alignment) or a local alignment (e.g., Smith-Waterman alignment).

Most attribute sorting algorithms can be characterized as one of three types based on their indexing method: algorithms based on hash tables (e.g., BLAST, ELAND, SOAP), suffix trees (e.g., Bowtie, BWA), and merge alignments (e.g., Slider). Short read sequences are typically used for alignment.

In the present invention, the alignment step in step (b) may be performed using, but not limited to, the BWA algorithm and the Hg19 sequence.

In the present invention, the BWA algorithm may include, but is not limited to, BWA-ALN, BWA-SW, or Bowtie2.

In the present invention, the length of the sequence information (reads) in step (b) is 5 to 5000 bp, and the number of used sequence information can be 50 to 5 million, but is not limited thereto.

In the present invention, the vectorized data of step (c) above can be any vectorized data that can be generated based on the aligned nucleic acid fragments, preferably a Grand Canyon plot (GC plot), but is not limited thereto.

In the present invention, the vectorized data can be preferably, but not exclusively, imaged. An image is essentially composed of pixels, which, when vectorized, can be represented as one-dimensional 2D vectors (black and white), three-dimensional 2D vectors (color (RGB)), or four-dimensional 2D vectors (color (CMYK)), depending on the type of image.

The vectorized data of the present invention is not limited to images, but for example, can be used as input data for an artificial intelligence model by stacking n black and white images into n multi-dimensional vectors.

In the present invention, a GC plot is a plot with a specific segment (either a constant size bin or a bin of different sizes) as the X-axis and a numerical value that can be expressed in terms of nucleic acid fragments, such as the distance or number between nucleic acid fragments, as the Y-axis.

The present invention further comprises the step of separately classifying nucleic acid fragments that satisfy a mapping quality score of the aligned nucleic acid fragments prior to performing step (c) above.

In the present invention, the mapping quality score may vary depending on the desired criteria, but may preferably be 15 to 70 points, more preferably 50 to 70 points, and most preferably 60 points.

In the present invention, the GC plot of step (c) above may be characterized that the distribution of the aligned nucleic acid fragments for each chromosomal segment is generated as vectorized data by counting the number of nucleic acid fragments for each position or the distance between nucleic acid fragments.

The method of vectorizing the number of nucleic acid fragments or distance calculations between nucleic acid fragments in the present invention is not limited to any known technique for vectorizing calculations.

In the present invention, calculating the chromosomal segmental distribution of the aligned sequence information as a number of nucleic acid fragments is performed, comprising the following steps:

i) dividing chromosomes into bins;
ii) determining the number of nucleic acid fragments aligned in each bin;

iii) normalization by dividing the number of nucleic acid fragments determined in each bin by the total number of nucleic acid fragments in the sample; and

iv) generating a GC plot with the order of each bin as the x-axis value and the normalized value calculated in step iii) above as the y-axis value.

In the present invention, calculating the distribution of the aligned sequence information by chromosomal segment as a distance between nucleic acid fragments may comprise the following steps:

i) dividing chromosomes into bins;

ii) calculating the Fragments Distance (FD) value between the nucleic acid fragments aligned in each bin;

iii) determining the representative value (RepFD) of the distance for each segment based on the distance values calculated for each segment;

iv) normalization by dividing the representative value calculated in step iii) above by the representative value of all nucleic acid fragment distance values; and

iv) generating a GC plot with the order of each bin as the x-axis value and the normalized value calculated in step iii) above as the y-axis value.

In the present invention, the GC plot can be used by aligning the GC plots of chromosomes 1 to 22 along the y-axis to generate one image, or by combining the images generated for 1 to 22 along the z-axis.

In the present invention, the representative value (RepFD) may be one or more values selected from the group consisting of, but not limited to, the sum, difference, product, mean, median, quantile, minimum, maximum, variance, standard deviation, median absolute deviation, coefficient of variation, reciprocal thereof, and combinations thereof of the distances between nucleic acid fragments.

In the present invention, the bin may be 1 Kb to 3 Gb, but is not limited thereto.

The present invention may further use the step of grouping the nucleic acid fragments, wherein the grouping criteria may be based on the adaptor sequences of the aligned nucleic acid fragments. The distance between the nucleic acid fragments can be calculated for the selected sequence information by separating the nucleic acid fragments into the forward aligned nucleic acid fragments and the reverse aligned nucleic acid fragments.

In the present invention, the FD value may be defined, for the obtained n nucleic acid fragments, as a distance between the cut-off values of the i-th nucleic acid fragment and one or more nucleic acid fragments selected from the i+1-th to the n-th nucleic acid fragments.

In the present invention, the FD value can be obtained by calculating, for the obtained n nucleic acid fragments, a distance between the cut-off value of a first nucleic acid fragment and the cut-off value of one or more nucleic acid fragments selected from the group consisting of a second nucleic acid fragment to a n-th nucleic acid fragment, and using one or more values selected from the group consisting of the sum, difference, product, mean, logarithm of the product, logarithm of the sum, median, quartile, minimum, maximum, variance, standard deviation, median absolute deviation, and coefficient of variation, and/or the reciprocal of one or more thereof, a weighted calculation, and statistics which are not limited thereto, but is not limited thereto.

In the present invention, the terms "one or more values" and "and/or the reciprocal of one or more thereof" are construed to mean that any one or two or more of the foregoing numerical values may be used in combination.

In the present invention, the term "the cut-off value of one or more nucleic acid fragments" may be a value where a random value is added to or substracted from the median value.

For the obtained n nucleic acid fragments, the FD value can be defined as follows:

$$FD = Dist(R_i \sim R_j) \ (1 < i < j < n),$$

wherein the Dist function calculates one or more values selected from the group consisting of the sum, difference, product, mean, logarithm of the product, logarithm of the sum, median, quartile, minimum, maximum, variance, standard deviation, median absolute deviation, and coefficient of variation of the difference in alignment position values of all nucleic acid fragments contained between the two selected $R_i$ and $R_j$ nucleic acid fragments, and/or the reciprocal of one or more thereof, a weighted calculation, and statistics which are not limited thereto.

In other words, in the present invention, FD value (Fragment Distance Value) means the distance between the aligned nucleic acid fragments. Here, the number of screening cases of nucleic acid fragments for distance calculation can be defined as follows. If there are a total of N nucleic acid fragments, there are $\sum_{k=1}^{n-1} k$ possible combinations of distances between nucleic acid fragments, i.e., if i is 1, i+1 is 2, and the distance to any one or more nucleic acid fragments selected from the 2nd to the n-th nucleic acid fragment can be defined.

In the present invention, the FD value may be obtained by calculating a distance between a particular position inside the i-th nucleic acid fragment and a particular position inside any one or more of i+1-th to n-th nucleic acid fragments.

For example, if a nucleic acid fragment is 50 bp long and aligned to position 4,183 on chromosome 1, the genetic position values that can be used to calculate the distance of this nucleic acid fragment are positions 4,183 to 4,232 on chromosome 1.

If a nucleic acid fragment which is 50 bp long is align to position 4,232 on chromosome 1, the genetic position values that can be used to calculate the distance of this nucleic acid fragment are positions 4,232 to 4,281 on chromosome 1, and the FD value between the two nucleic acid fragments can be 1 to 99.

If another adjacent 50 bp long nucleic acid fragment is aligned to position 4123 on chromosome 1, the genetic position values that can be used to calculate the distance of this nucleic acid fragment are positions 4,123 to 4,172 on chromosome 1, and the FD value between the two nucleic acid fragments is 61 to 159, and the FD value with the first example nucleic acid fragment is 12 to 110, and the FD value may be one or more values selected from the group consisting of the sum, difference, product, mean, logarithm of the product, logarithm of the sum, median, quartile, minimum, maximum, variance, standard deviation, median absolute deviation, and coefficient of variation of one value within the range of the two FD values, and/or the reciprocal of one or more thereof, a weighted calculation, and statistics which are not limited thereto, preferably the reciprocal of one value within the range of the two FD values, but is not limited thereto.

Preferably, in the present invention, the FD value may be a value where a random value is added to or substracted from the median value of the nucleic acid fragment.

In the present invention, the median of an FD is the value that is most centered when the calculated FD values are sorted in order of size. For example, if there are three values, such as 1, 2, and 100, 2 is the median because it is the most central. If there are an even number of FD values, the median is determined by averaging the two values in the center. For example, if there are FD values of 1, 10, 90 and 200, the median is 50, which is the average of 10 and 90.

In the present invention, any values may be used as long as they are indicative of the position of the nucleic acid fragment, but may preferably be 0 to 5 kbp or 0 to 300% of the length of the nucleic acid fragment, 0 to 3 kbp or 0 to 200% of the length of the nucleic acid fragment, 0 to 1 kbp or 0 to 100% of the length of the nucleic acid fragment, more preferably 0 to 500 bp or 0 to 50% of the length of the nucleic acid fragment, but are not limited thereto.

In the present invention, in the case of paired-end sequencing, the FD value may be derived based on the position values of the forward and reverse sequence information (reads).

For example, in a 50 bp long paired-end read pair, if the forward read aligns to position 4183 on chromosome 1 and the reverse read aligns to position 4349, the two ends of this nucleic acid fragment are 4183 and 4349, and the cut-off value that can be used for the nucleic acid fragment distance is 4183 to 4349. In the other pair of paired-end reads adjacent to the above nucleic acid fragment, if the forward read is aligned to the position 4349 of chromosome 1 and the reverse read is aligned to the position 4515, the position value of this nucleic acid fragment is 4349 to 4515. The distance between these two nucleic acid fragments can be 0 to 333, and most preferably 166, which is the median distance of each nucleic acid fragment.

The present invention may further comprise the step of excluding from the calculation process nucleic acid fragments having an alignment score of the sequence information (reads) below a cut-off value when the sequence information is obtained by paired-end sequencing.

In the present invention, the FD value may be derived based on one type of position value of the forward or reverse sequence information (read) in the case of single-end sequencing.

In the present invention, the single-end sequencing may be performed by adding an arbitrary value when deriving a position value based on forward aligned sequence information, and subtracting an arbitrary value when deriving a position value based on reverse aligned sequence information, wherein the arbitrary value can be any value that allows the FD value to clearly indicate the position of the nucleic acid fragment, but is preferably 0 to 5 kbp or 0 to 300% of the length of the nucleic acid fragment, 0 to 3 kbp or 0 to 200% of the length of the nucleic acid fragment, 0 to 1 kbp or 0 to 100% of the length of the nucleic acid fragment, more preferably 0 to 500 bp or 0 to 50% of the length of the nucleic acid fragment, but is not limited thereto.

The nucleic acids to be analyzed in the present invention can be sequenced and expressed in units called reads. These reads can be divided into single end sequencing (SE) and paired end sequencing (PE) reads depending on the sequencing method. An SE read is a sequence of a certain length of either the 5' or 3' of a nucleic acid molecule in a randomized direction, while a PE read is a sequence of a certain length of both the 5' and 3'. Because of this difference, it is well known to those of ordinary skill in the art that sequencing in SE mode produces one read from a single nucleic acid fragment, while sequencing in PE mode produces two reads in pairs from a single nucleic acid fragment.

The ideal way to calculate the exact distance between nucleic acid fragments would be to sequence the nucleic acid molecule from start to finish, align the reads, and use the median (center) of the aligned values. However, this is technically limited by the limitations and cost of sequencing technology. Therefore, sequencing is performed using SE or PE. In the case of PE, the start and end positions of the nucleic acid molecule are known, so the exact position (median) of the nucleic acid fragment can be determined through the combination of these values, but in the case of SE, only one end of the nucleic acid fragment is available, so there are limitations in calculating the exact position (median).

Also, when calculating the distance of nucleic acid molecules that are sequenced (aligned) in both directions, forward and reverse, using the terminal information of all reads, an inaccurate value may be calculated due to a factor called sequencing orientation.

Thus, for technical reasons of the sequencing method, the 5' end of the forward read has a position value that is smaller than the center of the nucleic acid molecule, while the 3' end of the reverse read has a larger value. Using this feature, it is possible to estimate a value close to the center of the nucleic acid molecule by adding an arbitrary value (Extended bp) to the forward read and subtracting arbitrary value (Extended bp) from the reverse read.

In other words, the arbitrary value (Extended bp) may vary depending on the sample used, and in the case of cell-free nucleic acids, it is known that the average length of such nucleic acids is about 166 bp, so it can be set to about 80 bp. If the experiment was performed using fragmentation equipment (e.g. sonication), about half of the target length set during the fragmentation process can be set as the extended bp.

In the present invention, the representative value (RepFD) may be one or more values selected from the group consisting of the sum, difference, product, mean, median, quartile, minimum, maximum, variance, standard deviation, median absolute deviation, and coefficient of variation of the FD values and/or the reciprocal of one or more thereof, preferably, the median, mean, or reciprocal of the FD values, but is not limited thereto.

In the present invention, the artificial intelligence model in step (d) can be any model that can be trained to distinguish between a normal image and a cancerous image, preferably a deep learning model.

In the present invention, the artificial intelligence model may be any artificial neural network algorithm capable of analyzing vectorized data based on an artificial neural network, but is preferably selected from the group consisting of a convolutional neural network (CNN), a deep neural network (DNN), a recurrent neural network (RNN), and an autoencoder, but is not limited thereto.

In the present invention, the recurrent neural network may be selected from the group consisting of a long-short term memory (LSTM) neural network, a gated recurrent unit (GRU) neural network, a vanilla recurrent neural network, and an attentive recurrent neural network.

In the present invention, when the artificial intelligence model is a CNN, the loss function for performing binary classification may be represented by following Formula 1, and the loss function for performing multi-class classification may be represented by the following Formula 2.

Formula 1: Binary classification

$$\text{loss}(\text{model}(x), y) = -\frac{1}{n}\left[\sum_{i=1}^{n}(y_i \log(model(x_i)) + (1 - y_i)\log(1 - model(x_i)))\right]$$

model $(x_i)$ = *Output of the artificial intelligence model for the $i_{th}$ input*
y = *Actual label value*
n = *Number of input data*

Formula 2: Multi-class classification

$$\text{loss}(\text{model}(x), y) = -\frac{1}{n}\sum_{i=1}^{n}(\sum_{j=1}^{c}(y_{ij} \log(model(x_i))_j)$$

model$(x_i)_j$ = *Output of the $j_{th}$ artifical intelligence model for the $i_{th}$ input*
y = *Actual label value*
n = *Number of input data*
c = *Number of class*

In the present invention, the binary classification refers to training an artificial intelligence model to determine the presence or absence of cancer, and the multi-class classification refers to training an artificial intelligence model to determine the type of cancer.

In the present invention, where the artificial intelligence model is a CNN, the training may be performed including the following steps:

i) categorizing the produced GC plots into training, validation, and test data, wherein training data is used to train the CNN model, validation data is used to validate hyper-parameter tuning, and test data is used for performance evaluation after producing the optimal model;
ii) building an optimal CNN model through hyper-parameter tuning and training process; and
iii) comparing the performance of multiple models obtained through hyper-parameter tuning using validation data, and determining the model with the best validation data performance as the optimal model.

In the present invention, the hyper-parameter tuning process is a process of optimizing the values of multiple parameters (number of convolutional layers, number of dense layers, number of convolutional filters, etc.) comprised of a CNN model, wherein the hyper-parameter tuning process can use Bayesian optimization and grid search techniques.

In the present invention, the learning process may be characterized by optimizing the internal parameters (weights) of the XGBoost model by using a set of hyper-parameters, and determining that the model is overfitted when the validation loss starts to increase compared to the training loss, and stopping the model training before that.

In the present invention, the resultant value of the analysis from the vectorized data with the artificial intelligence model input in step (d) may be any specific score or real number, preferably a Deep Probability Index (DPI) value, but is not limited thereto.

In the present invention, deep probability index means a value expressed as a probability value by adjusting the output of artificial intelligence to a scale of 0 to 1 using a sigmoid function for binary classification and a softmax function for multi-class classification in the last layer of the artificial intelligence model.

In the case of binary classification, the sigmoid function is used to train the DPI value to be 1 for cancer. For example, if a neuroblastoma sample and a normal sample are input, it is trained so that the DPI value of the neuroblastoma sample is closer to 1.

In the case of multi-class classification, the softmax function is used to select the DPI values equal to the number of classes. The sum of the DPI values equal to the number of classes becomes 1, and it is trained so that the DPI value of the actual cancer type becomes 1. For example, if there are 3 classes of neuroblastoma, liver cancer, and normal, and a neuroblastoma sample is input, the breast cancer class is trained to be close to 1.

In the present invention, the output of step (d) may be derived by cancer type.

In the present invention, when the artificial intelligence model is trained, the output result is trained to be close to 1 if there is cancer, and the output result is trained to be close to 0 if there is no cancer, and the performance measurement is performed by judging that there is cancer if the output result is above 0.5, and there is no cancer if the output result is below 0.5 (training, validation, test accuracy).

Here, it is apparent to one of ordinary skill in the art that the cut-off value of 0.5 is a value that can be changed at any time. For example, if it is desired to reduce false positives, the cut-off value higher than 0.5 can be set to set strict criteria for determining if there is cancer, and if it is desired to reduce false negatives, the lower cut-off value can be set to set loose criteria for determining if there is cancer.

Most preferably, a trained artificial intelligence model can be used to apply unseen data (data that knows the answer which was not trained) to determine the probability of an XPI value to establish a cut-off value.

In the present invention, the step (e) of predicting a cancer type by comparing the output result values may comprise the step of determining the cancer type representing the highest value among the output result values as a cancer of the sample.

In another aspect, the present invention provides a cancer diagnosis and cancer type prediction device comprising:

a decoding part that extracts nucleic acid from a biospecimen and decodes sequence information including methylation information;
an alignment part that aligns the decoded sequence to a reference genome database;
a data generation part that generates vectorized data using nucleic acid fragments based on the aligned sequence;
a cancer diagnosis part that inputs the generated vectorized data into a trained artificial intelligence model, analyzes same, and determines the presence or absence of cancer by comparing the analyzed output value to a cut-off value; and
a cancer type prediction part that analyzes the output result value to predict a cancer type.

In the present invention, the decoding part may be performed in an independent device. For example, the decoding part of the present invention may produce sequence information, i.e., reads, containing methylation information, in an NGS device.

In another aspect, the present invention provides a computer-readable storage medium comprising instructions configured to be executed by a processor for diagnosing cancer and predicting a cancer type, the instructions comprising:

(a) obtaining a sequence information including methylation information from extracted nucleic acids from a biospecimen;
(b) aligning the obtained sequence information (reads) to a reference genome database;
(c) generating vectorized data using nucleic acid fragments based on the aligned sequence information (reads);
(d) inputting the generated vectorized data into a trained artificial intelligence model and comparing the analyzed output value with a cut-off value to determine the presence or absence of cancer; and
(e) predicting a cancer type through the comparison with the output result.

The present invention provides, in another aspect, a method for providing information for diagnosing cancer and predicting cancer type, comprising the steps of:

(a) obtaining a sequence information including methylation information from extracted nucleic acids from a biospecimen;
(b) aligning the obtained sequence information (reads) to a reference genome database;
(c) generating vectorized data using nucleic acid fragments based on the aligned sequence information (reads);
(d) inputting the generated vectorized data into a trained artificial intelligence model and comparing the analyzed output value with a cut-off value to determine the presence or absence of cancer; and
(e) predicting a cancer type through the comparison with the output result.

In another aspect, the method according to the present invention may be implemented by using a computer. In one embodiment, the computer includes one or more processors coupled to a chip set. Also connected to the chip set are memory, storage devices, a keyboard, a graphics adapter, a pointing device, and a network adapter. In one embodiment, the performance of the chip set is enabled by a memory controller hub and an I/O controller hub. In other embodiment, the memory may be directly connected to a processor instead of a chip set. The storage device is any device capable of holding data, including a hard drive, compact disk read-only memory (CD-ROM), DVD, or other memory device. The memory is involved in the data and instructions used by the processor. The pointing device may be a mouse, track ball, or other type of pointing device, and is used in combination with a keyboard to transmit input data to the computer system. A graphics adapter represents images and other information on a display. The network adapter is connected to the computer system by a local area or long distance communication network. The computers used herein are not limited to the above configurations, however, and may include some or additional configurations, and may also be part of a storage area network (SAN), and the computers may be configured to be suitable for executing modules in a program for performing the methods according to the present invention.

As used herein, a module may refer to a functional and structural combination of hardware for performing a technical idea according to the present invention and software for operating the hardware. For example, it is apparent to a person skilled in the art that a module may refer to a logical unit of predetermined code and a hardware resource on which the predetermined code is performed, and does not necessarily refer to physically connected code or a type of hardware.

Methods according to the present invention may be implemented in hardware, firmware, or software, or a combination thereof. When implemented as software, the storage medium includes any medium for storing or communicating information in a form readable by a device such as a computer. For example, computer-readable media include read only memory (ROM); random access memory (RAM); magnetic disk storage media; optical storage media; flash memory devices; and other electrical, optical, or acoustic signal transmission media.

## Example

The present invention is described in more detail by providing the following examples. These examples are intended solely to illustrate the present invention, and it would be apparent to one of ordinary skill in the art that the scope of the present invention is not to be construed as limited by these examples.

## Example 1. Extracting methylated cfDNA from blood and performing next-generation sequencing

Blood from 185 normal individuals and 57 patients with neuroblastoma was collected and subjected to primary centrifugation of only the plasma at 3000 rpm, at 25°C, for 10 minutes, and then the primary centrifuged plasma was subjected to secondary centrifugation in an amount of 16000g, at 25°C, for 10 minutes to separate the plasma supernatant excluding sediment. For the separated plasma, cell-free DNA was extracted using chemagen DNA kit, followed by adaptor ligation using Truseq Nano DNA HT library prep kit (Illumina), followed by 5mC immunoprecipitation using antibodies from cfMedilP kit (diagnode) at 10 rpm, at 4°C, for 17 hours, followed by purification, followed by PCR enrichment using Truseq Nano DNA HT library prep kit (Illumina), and finally a library was prepared. The produced library was sequenced using Novaseq 6000 (Illumina) in 150 paired-end mode, yielding approximately 30 million reads per sample.

**Example 2. Generating a GC plot based on the number of nucleic acid fragment counts**

The reads obtained in Example 1 were aligned to the reference genome using the bwa (version 0.7.17-r1188) alignment tool, then PCR duplicate nucleic acid fragments were removed using the biobambam2 bammarkduplicates (version 2.0.87) tool, and nucleic acid fragments with a mapping quality of 60 or less were removed using sambamba (version 0.6.6).

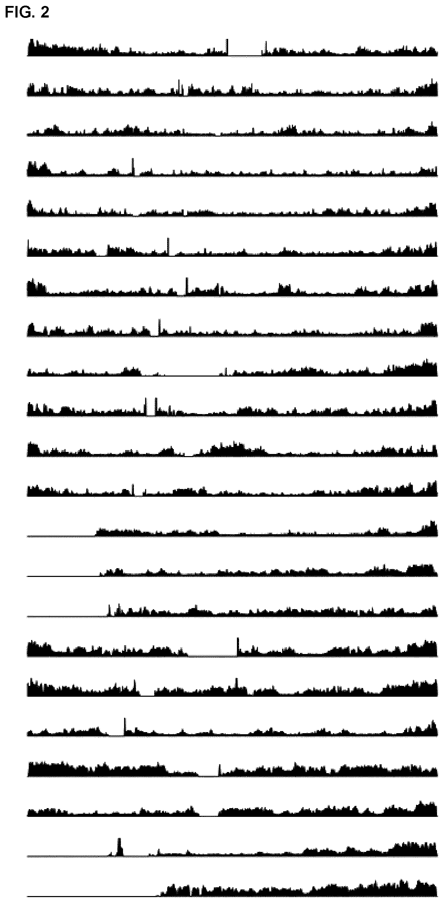

GC plot represents the alignment of NGS reads from the beginning to the end of a chromosome. All chromosomes, except the sex chromosomes, were divided into nonoverlapping 100 kilobase bins, and the number of reads assigned to each bin was counted (read count value). The read count value assigned to each bin was then divided by the total number of reads in the sample for normalization. With the normalized bin read count value as Y value and the order of each bin as X value, GC plots were produced for each chromosome, and the produced GC plots were aligned in chromosome 1 to chromosome 22 to produce one image (FIG. 2).

**Example 3. Building a GC plot-based neuroblastoma deep learning model and calculating DPI**

3-1. Building a deep learning model

The GC plots produced in Example 2 were divided into training, validation, and test data. The training data was used to train the CNN model, the validation data was used to validate hyper-parameter tuning, and the test data was used for performance evaluation after producing the optimal model.

Tensorflow (version 2.4.1) was used to build and train the CNN model, which has the following structure: convolution layer -> pooling layer -> fully connected layer, wherein a pooling layer is always inserted after the convolution layer. The number of convolutional layers and the number of fully connected layers were determined through hyper-parameter tuning. When training the model, it was trained to minimize the loss function, which is shown in Formulae 1 and 2.

To obtain the optimal model, hyper-parameter tuning was performed using the scikitoptimize (version 0.7.4) python package. Hyper-parameter tuning is a process of optimizing the values of various parameters (number of convolution layers, number of dense layers, number of convolution filters, etc.) that consist of a CNN model. Convolution layer number, convolution filter number, convolution patch size, fully connected layer number, hidden node number, activation function, dropout presence, and learning rate were specified as hyper-parameters, and the optimal model was built using Bayesian optimization technique. When training the model with the specified hyper-parameters, if the validation loss starts to increase compared to the training loss, the model was judged to be overfitting and the model learning is stopped before that. After comparing the performance of several models obtained during the hyper-parameter tuning process using the validation data, the model with the best performance was judged to be the optimal model, and the performance was performed using the test data.

3-2. Calculating deep probability index (DPI)

When data (GC plot) was input to the optimal model obtained through hyper-parameter tuning, the probability value was output through the output layer of the model.

First, for binary classification, a sigmoid function was used to the output layer of the model. The sigmoid function is shown in Formula 3 below:

$$\text{Formula 3 Sigmoid function: } \frac{1}{1+e^{-x}},$$

$x$ = output layer

The probability value (DPI) output from Formula 3 is one, which was used to diagnose cancer.

For multi-class classification, the softmax function was used to the output layer of the model, as shown in Formula 4:

Formula 4 Softmax function: $\dfrac{e^{x_i}}{\sum_{j=1}^{C} e^{x_j}}$,

C = Number of class,      $x_i$ = output layer in the $i_{th}$ class

The probability value (DPI) output in Formula 4 was equal to the number of classes, which was used to classify the cancer types.

**Example 4. Building a deep learning model for neuroblastoma and verifying performance of GC plots using methylated cfDNA based on the number of nucleic acid fragment**

The performance of DPI values was tested by using normal samples (n=186) and neuroblastoma samples (n=57). All samples were divided into Train, Validation, and Test groups, and the model was built using the Train sample, then the Validation and Test groups were used to check the performance of the model built using the Train sample.

[Table 1]

|  | Normal | Neuroblastoma | Total |
|---|---|---|---|
| Train | 130 | 39 | 169 |
| Valid | 28 | 9 | 37 |
| Test | 28 | 9 | 37 |
| Total | 186 | 57 | 243 |

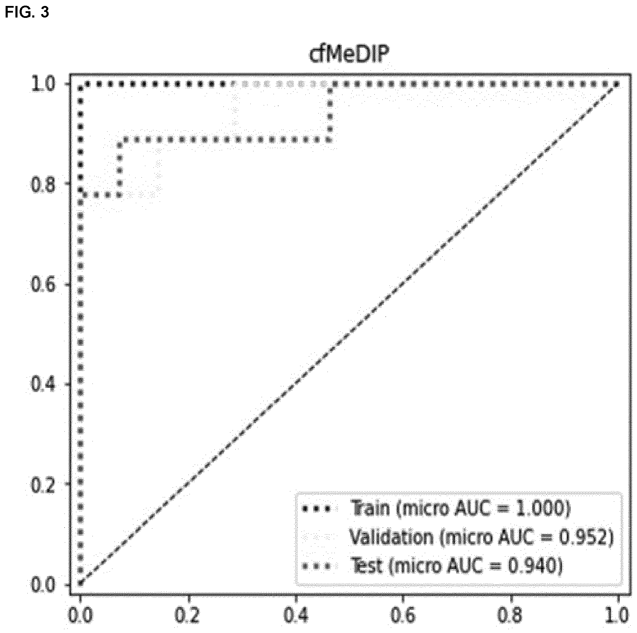

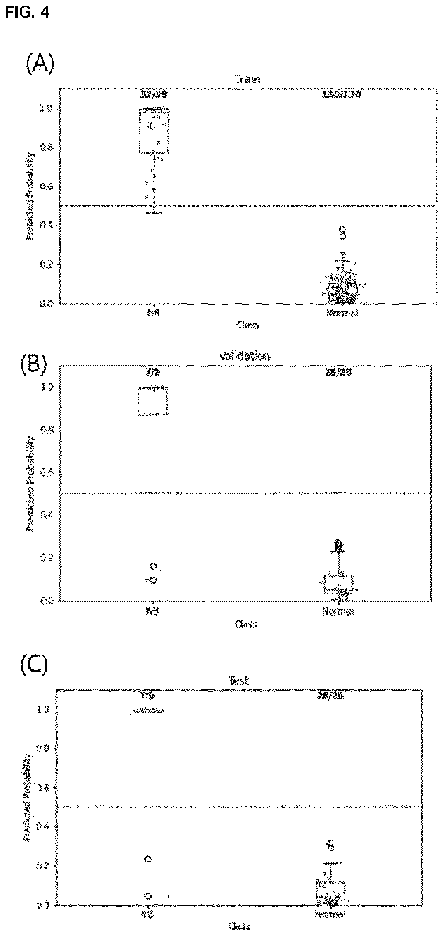

As a result, as shown in Table 2, and FIGs. 3 and 4, accuracy was found to be 100%, 92%, and 94.1% for Train, Valid, and Test groups, respectively, and AUC values were found to be 1.0, 0.95, and 0.99 for Train, Valid, and Test groups, respectively, based on ROC analysis.

FIG. 3 shows an analysis using a receiver operating characteristic (ROC) curve as a way to measure accuracy, where a higher area under the curve (AUC) value is interpreted as more accurate. The AUC value can be 0 to 1, with a (baseline) expected AUC value of 0.5 for randomly predicting a label value and an expected AUC value of 1 for perfectly accurate prediction.

FIG. 4 is a boxplot of the probability value of having cancer (DPI) calculated by the artificial intelligence model of the present invention in groups of normal and neuroblastoma samples, with the red line representing the DPI cutoff of 0.5.

[Table 2]

|  | Accuracy | AUC |
|---|---|---|
| Train | 98.8 | 1.0 |
| Valid | 94.6 | 0.95 |
| Test | 94.6 | 0.94 |

**Example 5. Building a deep learning model for neuroblastoma and verifing performance of GC plots using cfDNA based on the number of nucleic acid fragment**

5-1. Extracting DNA from blood for next-generation sequencing

10 ml of Blood from 186 healthy individuals and 57 neuroblastoma patients was stored in EDTA tubes, and within 2 hours after collection, only the plasma portion was subjected to primary centrifugation in an amount of 1200g, at 4°C, for 15 minutes, and then the primary centrifuged plasma was subjected to secondary centrifugation in an amount of 16000g, at 4°C, for 10 minutes to separate the plasma supernatant excluding sediment. For the separated plasma, cell-free DNA was extracted using Tiangenmicro DNA kit (Tiangen), library preparation was performed using MGIEasy cell-free DNA library prep set kit, and then sequencing using DNBseq G400 instrument (MGI) in 100 base paired end mode was performed. As a result, it was found that about 1.7 million reads were produced per sample.

5-2. Building a deep learning model and checking its performance

The performance of DPI values was tested by using normal samples (n=186) and neuroblastoma samples (n=57). All samples were divided into Train, Validation, and Test groups, and the model was built using the Train sample, then the Validation and Test groups were used to check the performance of the model built using the Train sample.

[Table 3]

|  | Normal | Neuroblastoma | Total |
|---|---|---|---|
| Train | 130 | 39 | 169 |
| Valid | 28 | 9 | 37 |
| Test | 28 | 9 | 37 |
| Total | 186 | 57 | 243 |

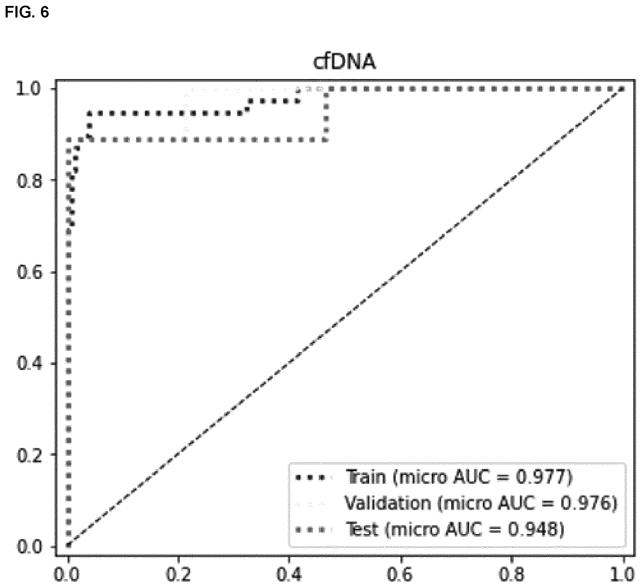

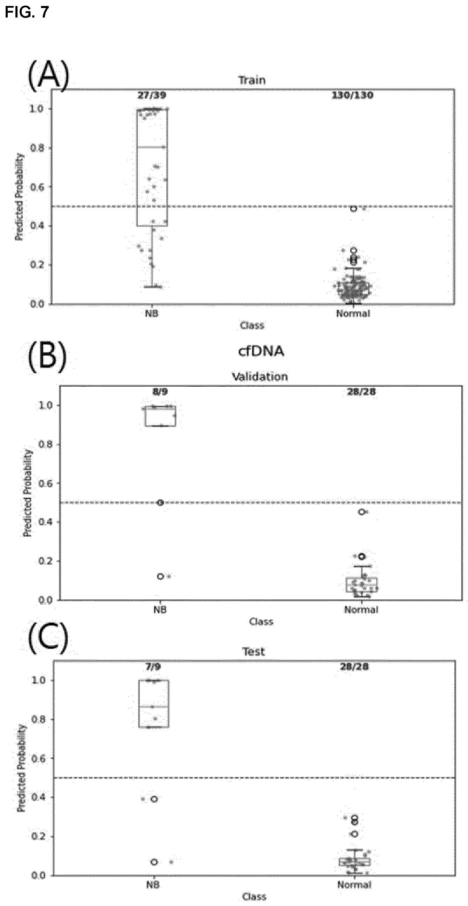

As a result, as shown in Table 4, and FIGs. 6 and 7, the accuracy was 92.9%, 97.3%, and 94.6% for the Train, Valid, and Test groups, respectively, and the ROC analysis showed that the AUC values were 0.98, 0.98, and 0.95 for the Train, Valid, and Test groups, respectively.

[Table 4]

|  | Accuracy | AUC |
|---|---|---|
| Train | 92.9 | 0.98 |
| Valid | 97.3 | 0.98 |
| Test | 94.6 | 0.95 |

While the foregoing has described in detail certain aspects of the present invention, it would be apparent to one of ordinary skill in the art that these specific descriptions are merely preferred embodiments and are not intended to limit the scope of the present invention. Accordingly, the substantial scope of the present invention is defined by the appended claims and their equivalents.

**Industrial Availability**

The method for diagnosing cancer and predicting cancer types using methylated cell-free nucleic acids according to the present invention is useful because it generates vectorized data and analyzes it using an AI algorithm, compared to methods that use a conventional step of determining the amount of chromosomes based on the read count or detection methods that use the concept of distance between aligned reads to utilize values related to reads as one by one structured values, so that a similar effect can be achieved even if the read coverage is low.

**Claims**

1.   A method for providing information for diagnosing cancer and predicting cancer type, comprising the steps of:

(a) obtaining a sequence information including methylation information from extracted nucleic acids from a biospecimen;
(b) aligning the obtained sequence information (reads) to a reference genome sequence database;
(c) generating vectorized data using nucleic acid fragments based on the aligned sequence information (reads);
(d) inputting the generated vectorized data into a trained artificial intelligence model and comparing the analyzed output value with a cut-off value to determine the presence or absence of cancer; and
(e) predicting a cancer type through the comparison with the output result.

2.   A method for diagnosing cancer and predicting cancer type, comprising the steps of:

(a) obtaining a sequence information including methylation information from extracted nucleic acids from a biospecimen;

(b) aligning the obtained sequence information (reads) to a reference genome sequence database;

(c) generating vectorized data using nucleic acid fragments based on the aligned sequence information (reads);

(d) inputting the generated vectorized data into a trained artificial intelligence model and comparing the analyzed output value with a cut-off value to determine the presence or absence of cancer; and

(e) predicting a cancer type through the comparison with the output result.

3. The method according to claim 1 or 2, wherein step (a) is performed by a method comprising the steps of:

(a-i) obtaining nucleic acids with methylation information from a biospecimen;

(a-ii) removing proteins, fats, and other residues from the collected nucleic acid by using a salting-out method, a column chromatography method, or a beads method to obtain purified nucleic acid;

(a-iii) creating a single-end sequencing or pair-end sequencing library from purified nucleic acids or nucleic acids that have been randomly fragmented by enzymatic cleavage, grinding, or hydroshear methods;

(a-iv) reacting the created library to a next-generation sequencer; and

(a-v) acquiring nucleic acid sequence information (reads) from a next-generation sequencer.

4. The method according to claim 3, wherein the methylation information of step (a-i) is obtained by bisulfite conversion, enzymatic conversion, or methylated DNA immunoprecipitation (MeDIP).

5. The method according to claim 1, wherein the vectorized data of step (c) is a grand canyon plot (GC plot).

6. The method according to claim 5, wherein the GC plot generates vectorized data by calculating a chromosomal segmental distribution of the aligned nucleic acid fragments as count per segment or distances between nucleic acid fragments.

7. The method according to claim 6, wherein calculating the chromosomal segmental distribution as a number of nucleic acid fragments is performed, comprising the following steps:

i) dividing chromosomes into bins;

ii) determining the number of nucleic acid fragments aligned in each bin;

iii) normalization by dividing the number of nucleic acid fragments determined in each bin by the total number of nucleic acid fragments in the sample; and

iv) generating a GC plot with the order of each bin as the x-axis value and the normalized value calculated in step iii) above as the y-axis value.

8. The method according to claim 6, wherein calculating the chromosomal segmental distribution as a distance between nucleic acid fragments, comprising the following steps:

i) dividing chromosomes into bins;

ii) calculating the Fragments Distance (FD) value between the nucleic acid fragments aligned in each bin;

iii) determining the representative value (RepFD) of the distance for each segment based on the distance values calculated for each segment;

iv) normalization by dividing the representative value calculated in step iii) above by the representative value of all nucleic acid fragment distance values; and

iv) generating a GC plot with the order of each bin as the x-axis value and the normalized value calculated in step iii) above as the y-axis value.

9. The method according to claim 8, wherein the representative value is at least one selected from the group consisting of the sum, difference, product, mean, median, quartile, minimum, maximum, variance, standard deviation, median absolute deviation, coefficient of variation, reciprocal values thereof, and combinations thereof of the distances between nucleic acid fragments.

10. The method according to claim 1, wherein the artificial intelligence model of step (d) is trained to distinguish between vectorized data that is normal and vectorized data that has cancer.

11. The method according to claim 10, wherein the artificial intelligence model is selected from the group consisting of a convolutional neural network (CNN), a deep neural network (DNN), a recurrent neural network (RNN), and an autoencoder.

12. The method according to claim 10, wherein when the artificial intelligence model is a CNN, the loss function for performing binary classification may be represented by following Formula 1, and the loss function for performing multi-class classification may be represented by the following Formula 2:

Formula 1: Binary classification

$$\text{loss(model(x),y)} = -\frac{1}{n}\left[\sum_{i=1}^{n}\left(y_i \log\big(model(x_i)\big) + (1 - y_i)\log\big(1 - model(x_i)\big)\right)\right]$$

model($x_i$) = *Output of the artificial intelligence model for the $i_{th}$ input*
y = Actual label value
n = Number of input data

Formula 2: Multi-class classification

$$\text{loss(model(x),y)} = -\frac{1}{n}\sum_{i=1}^{n}\left(\sum_{j=1}^{c}\left(y_{ij}\log(model(x_i))_j\right)\right)$$

model($x_i)_j$ = *Output of the $j_{th}$ artifical intelligence model for the $i_{th}$ input*
y = Actual label value
n = Number of input data
c = Number of class

13. The method according to claim 1, wherein the output value of the analysis from the vectorized data with the artificial intelligence model input in step (d) is a deep probability index (DPI) value.

14. The method according to claim 1, wherein the cut-off value of step (d) is 0.5, and a value greater than 0.5 determines that the patient has cancer.

15. The method according to claim 1, wherein the step (e) of predicting a cancer type by comparing the output result values may comprise the step of determining the cancer type representing the highest value among the output result values as a cancer of the sample.

16. A cancer diagnosis and cancer type prediction device comprising:

a decoding part that extracts nucleic acid from a biospecimen and decodes sequence information including methylation information;
an alignment part that aligns the decoded sequence to a reference genome database;
a data generation part that generates vectorized data using nucleic acid fragments based on the aligned sequence;
a cancer diagnosis part that inputs the generated vectorized data into a trained artificial intelligence model, analyzes same, and determines the presence or absence of cancer by comparing the analyzed output value to a cut-off value; and
a cancer type prediction part that analyzes the output result value to predict a cancer type.

17. A computer-readable storage medium comprising instructions configured to be executed by a processor for diagnosing cancer and predicting a cancer type, the instructions comprising:

(a) obtaining a sequence information including methylation information from extracted nucleic acids from a biospecimen;
(b) aligning the obtained sequence information (reads) to a reference genome database;

(c) generating vectorized data using nucleic acid fragments based on the aligned sequence information (reads);

(d) inputting the generated vectorized data into a trained artificial intelligence model and comparing the analyzed output value with a cut-off value to determine the presence or absence of cancer; and

(e) predicting a cancer type through the comparison with the output result.

FIG. 1

```
┌─────────────────────────────────────────────────────┐
│                                                       │
│     Concentrating a methylated cell-free nucleic acid│
│                                                       │
└─────────────────────────────────────────────────────┘
                          │
                          ▼
┌─────────────────────────────────────────────────────┐
│     Acquiring concentrated nucleic acid fragments    │
│     by using massively parallel sequencing method    │
└─────────────────────────────────────────────────────┘
                          │
                          ▼
┌─────────────────────────────────────────────────────┐
│        Aligning fragment data to human reference     │
│                      chromosome                      │
└─────────────────────────────────────────────────────┘
                          │
                          ▼
┌─────────────────────────────────────────────────────┐
│          Determining quality of aligned data         │
└─────────────────────────────────────────────────────┘
                          │
                          ▼
┌─────────────────────────────────────────────────────┐
│          Generating a GC (Grand Canyon) plot         │
└─────────────────────────────────────────────────────┘
                          │
                          ▼
┌─────────────────────────────────────────────────────┐
│      Training CNN (convolution neural network)       │
└─────────────────────────────────────────────────────┘
                          │
                          ▼
┌─────────────────────────────────────────────────────┐
│        Calculating deep probability index (DPI)      │
└─────────────────────────────────────────────────────┘
                          │
                          ▼
┌─────────────────────────────────────────────────────┐
│   Predicting whether the subject is a cancer patient │
│                and the type of cancer                │
└─────────────────────────────────────────────────────┘
```

FIG. 2

FIG. 3

FIG. 4

(A)

(B)

(C)

FIG. 5

FIG. 6

cfDNA

Train (micro AUC = 0.977)
Validation (micro AUC = 0.976)
Test (micro AUC = 0.948)

FIG. 7

(A) Train

(B) cfDNA

(C) Test

## INTERNATIONAL SEARCH REPORT

| International application No. |
|---|
| **PCT/KR2022/016448** |

| A. | CLASSIFICATION OF SUBJECT MATTER |
|---|---|

**G16B 5/00**(2019.01)i; **G16B 35/10**(2019.01)i; **G16B 30/10**(2019.01)i; **G16B 40/00**(2019.01)i; **G06N 3/04**(2006.01)i; **G06N 3/08**(2006.01)i; **C12Q 1/6886**(2018.01)i

According to International Patent Classification (IPC) or to both national classification and IPC

| B. | FIELDS SEARCHED |
|---|---|

Minimum documentation searched (classification system followed by classification symbols)

G16B 5/00(2019.01); C12Q 1/68(2006.01); G16B 10/00(2019.01); G16B 15/00(2019.01); G16B 20/00(2019.01); G16B 20/20(2019.01); G16B 30/00(2019.01); G16B 30/10(2019.01); G16B 40/20(2019.01); G16B 50/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 메틸화(methylation), 샘플(sample), 핵산(nucleic acid), 서열(sequence), 표준 염색체 서열 데이터베이스(reference genome database), 정렬(alignment), 단편(fragment), 벡터화(vector), 인공지능(artificial intelligent), 학습(learning), 기준값(cut-off), 암(cancer), 암종(carcinoma), 진단(diagnosis), 예측(prediction)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | KR 10-2021-0067931 A (GREEN CROSS GENOME CORPORATION) 08 June 2021 (2021-06-08) See abstract, claims 1-7 and 12-18, paragraphs [0003], [0043], [0046]-[0051] and [0092], and equations 2-3. | 1-17 |
| Y | KR 10-2015-0082228 A (THE CHINESE UNIVERSITY OF HONG KONG) 15 July 2015 (2015-07-15) See claims 1 and 6, and paragraphs [0011], [0048], [0077], [0105]-[0106], [0320], [0527]-[0528] and [0561]. | 1-17 |
| A | KR 10-2019-0085667 A (GREEN CROSS GENOME CORPORATION) 19 July 2019 (2019-07-19) See entire document. | 1-17 |
| A | KR 10-2019-0036494 A (EWHA UNIVERSITY - INDUSTRY COLLABORATION FOUNDATION) 04 April 2019 (2019-04-04) See entire document. | 1-17 |

☑ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **06 February 2023** | **06 February 2023** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| | International application No. |
|---|---|
| | **PCT/KR2022/016448** |

**C.      DOCUMENTS CONSIDERED TO BE RELEVANT**

| Category* | Citation of document, with indication, where appropriate, of the relevant passages | Relevant to claim No. |
|---|---|---|
| A | KR 10-2021-0021923 A (GREEN CROSS GENOME CORPORATION) 02 March 2021 (2021-03-02)<br>See entire document. | 1-17 |
| PX | KR 10-2022-0074088 A (GC GENOME CORPORATION et al.) 03 June 2022 (2022-06-03)<br>See claims 1-15. | 1-3,5-17 |

Form PCT/ISA/210 (second sheet) (July 2022)

International application No.

**PCT/KR2022/016448**

| Patent document cited in search report | | | Publication date (day/month/year) | Patent family member(s) | | | Publication date (day/month/year) |
|---|---|---|---|---|---|---|---|
| KR | 10-2021-0067931 | A | 08 June 2021 | EP | 4068291 | A1 | 05 October 2022 |
| | | | | WO | 2021-107676 | A1 | 03 June 2021 |
| KR | 10-2015-0082228 | A | 15 July 2015 | CN | 104781422 | A | 15 July 2015 |
| | | | | EP | 2898100 | A1 | 29 July 2015 |
| | | | | EP | 2898100 | B1 | 22 November 2017 |
| | | | | EP | 3354747 | A1 | 01 August 2018 |
| | | | | EP | 3354747 | B1 | 17 February 2021 |
| | | | | EP | 3536807 | A1 | 11 September 2019 |
| | | | | EP | 3839065 | A1 | 23 June 2021 |
| | | | | EP | 4056712 | A1 | 14 September 2022 |
| | | | | JP | 2015-536639 | A | 24 December 2015 |
| | | | | JP | 2018-121644 | A | 09 August 2018 |
| | | | | JP | 2020-108400 | A | 16 July 2020 |
| | | | | JP | 2022-031753 | A | 22 February 2022 |
| | | | | JP | 6317354 | B2 | 25 April 2018 |
| | | | | JP | 6683752 | B2 | 22 April 2020 |
| | | | | JP | 6985753 | B2 | 22 December 2021 |
| | | | | KR | 10-2020-0085928 | A | 15 July 2020 |
| | | | | KR | 10-2021-0120124 | A | 06 October 2021 |
| | | | | KR | 10-2022-0065034 | A | 19 May 2022 |
| | | | | KR | 10-2148547 | B1 | 26 August 2020 |
| | | | | KR | 10-2307424 | B1 | 29 September 2021 |
| | | | | KR | 10-2390711 | B1 | 26 April 2022 |
| | | | | US | 10392666 | B2 | 27 August 2019 |
| | | | | US | 10706957 | B2 | 07 July 2020 |
| | | | | US | 11274347 | B2 | 15 March 2022 |
| | | | | US | 2014-0080715 | A1 | 20 March 2014 |
| | | | | US | 2015-0011403 | A1 | 08 January 2015 |
| | | | | US | 2018-0105884 | A1 | 19 April 2018 |
| | | | | US | 2019-0241979 | A1 | 08 August 2019 |
| | | | | US | 2020-0388349 | A1 | 10 December 2020 |
| | | | | US | 9732390 | B2 | 15 August 2017 |
| | | | | WO | 2014-043763 | A1 | 27 March 2014 |
| KR | 10-2019-0085667 | A | 19 July 2019 | KR | 10-2029393 | B1 | 07 October 2019 |
| | | | | WO | 2019-139363 | A1 | 18 July 2019 |
| KR | 10-2019-0036494 | A | 04 April 2019 | KR | 10-2233740 | B1 | 30 March 2021 |
| | | | | WO | 2019-066421 | A2 | 04 April 2019 |
| | | | | WO | 2019-066421 | A3 | 04 July 2019 |
| KR | 10-2021-0021923 | A | 02 March 2021 | EP | 4020484 | A1 | 29 June 2022 |
| | | | | JP | 2022-544626 | A | 19 October 2022 |
| | | | | KR | 10-2452413 | B1 | 11 October 2022 |
| | | | | WO | 2021-034034 | A1 | 25 February 2021 |
| KR | 10-2022-0074088 | A | 03 June 2022 | WO | 2022-114631 | A1 | 02 June 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10975431 B
- US 20200131582 A
- KR 102148547
- KR 102018124550
- KR 1020197038076
- KR 1020190003676
- KR 1020190001741
- KR 1020210067931

**Non-patent literature cited in the description**

- **ZHOU, XIONGHUI et al.** *bioRxiv*
- **LI, JIAQI et al.** *bioRxiv*
- **HINTON ; GEOFFREY et al.** *IEEESignal Processing Magazine,* 2012, vol. 29 (6), 82-97
- **METZKER, M.** *Nature Biotechnology Reviews,* 2010, vol. 11, 31-46